# EUROPEAN PATENT SPECIFICATION

(11) **EP 1 267 858 B1**
(45) Date of publication and mention of the grant of the patent: **28.07.2004**
(21) Application number: 01938038.5
(22) Date of filing: 23.03.2001
(51) Int. Cl.: A61K 31/12, A61K 31/196, A61P 31/18

(54) **DIPHENYL KETOALDEHYDE DERIVATIVES WITH ANTI-HIV ACTIVITY**
DIPHENYL KETOALDEHYDE-DERIVATE MIT WIRKUNG GEGEN HIV
DERIVES DE DIPHENYL KETOALDEHYDE POSSEDANT UNE ACTIVITE ANTI-VIH

(30) Priority: 24.03.2000 IT MI000629
(43) Date of publication of application: 02.01.2003
(73) Proprietor: Unihart Corporation, Dublin 2 (IE)
(72) Inventor: TARRO, Giulio, I-80123 Napoli (IT)
(74) Representative: Minoja, Fabrizio, Dr.
(86) International application number: PCT/EP2001/003343
(87) International publication number: WO 2001/070216

(56) References cited:
- US-A- 5 147 652
- HAZUDA D J ET AL: "Inhibitors of strand transfer that prevent integration and inhibit HIV - 1 replication in cells." SCIENCE, (2000 JAN 28) 287 (5453) 646-50. , XP002175834
- MAGRASSI, F. ET AL: "Mechanism of antiviral activity of biphenyl ketoaldehydes" PROC. CONGR. HUNG. ASS. MICROBIOL., 4TH, BUDAPEST (1966), VOLUME DATE 1964 11-20, XP001013381
- MAGRASSI, F. ET AL: "The mechanism of action of a group of antiviral drugs (biphenyl ketoaldehydes and their derivatives), and factors which interfere with their protective activity in vivo" INTERN. CONGR. CHEMOTHERAPY, PROC., 3RD, STUTTGART (1964), 1963(1), 783-95, XP001017961
- ALTUCCI, PAOLO ET AL: "Mechanism of action of biphenyl ketoaldehyde derivatives" CHEMOTHERAPY (1969), 14(2), 86-92 , XP001013474
- TARRO G. ET AL: "[Some biological and immunologic characteristics of the virus of murine hepatitis inactivated in vitro by xenygloxal ]. SU ALCUNE CARATTERISTICHE BIOLOGICHE ED IMMUNOLOGICHE DEL VIRUS DELL'EPATITE MURINA (MHV3) INATTIVATO IN VITRO DALLO XENALDIALE." RASSEGNA DI MEDICINA SPERIMENTALE, (1975) 22/2-3 (81-89). CODEN: RMSPAY, XP001013519

## Description

The present invention relates to the treatment of viral infections. More particularly, the invention relates to the use of molecules known for their antiviral activity against influenza virus, for the treatment of infections caused by virus responsible for human immune deficiency (HIV).

Retrovirus HIV is the etiological agent of a complex disease that induces the progressive destruction of the immune system (acquired immune deficiency syndrome; AIDS) and the degeneration of the central and peripheral nervous system.

Retroviruses differ from the other viruses in two aspects of the viral replication cycle: 1) reverse transcription resulting in the production of a double stranded cDNA copy by the viral RNA genome, and 2) integration of cDNA in the host DNA. Integration is a multi-stage process comprising the assembly of a stable complex between the integrase and specific viral DNA sequences, the subsequent endonucleolytic processing to remove the terminal dinucleotide from each 3' end of the viral DNA and the strand transfer in which the viral DNA 3' ends are covalently linked to the cellular DNA. The integration mechanism is at present extensively studied in order to develop novel compounds for use in the treatment of HIV infections *(1-6)*. Particularly interesting is the finding of integrase inhibitors active against HIV in cell cultures. In this respect, Hazuda reported the identification of novel integrase inhibitors whose antiviral activity is directly related to their effect on integration *(7)*.

Diphenyl ketoaldehyde derivatives are a group of molecules known for their antiviral activity *(8)*. In particular, the compounds xenalamine (p-(α-ethoxy-p-phenylphenacyl-amino)benzoic acid) and xenaldial (4-4'-bis-biphenylglyoxal dihydrate) proved to be active against A-PR8 Influenza virus *(9)*, MHV-3 hepatitis virus and various Arborvirus, Poxvirus, Nitavirus and Myxovirus strains.

The inventors have carried out extensive searches for novel compounds having inhibiting activity on viral integrase, and they found that some diphenyl ketoaldehyde derivatives are capable of reducing and in some cases even arresting the growth of the HIV virus in cultured infected cells. Among the various tested compounds, xenalamine, xenaldial and xenalic acid showed a marked virucidal activity and, to a lower extent, inhibiting activity on viral replication *in vitro.* Particularly effective turned out to be the compound xenaldial.

Therefore, in a first aspect, the invention relates to the use of xenalamine, xenalic acid and, preferably, xenaldial, for the preparation of a medicament for the treatment of HIV infections.

In the *in vitro* cultures used to assay the activity of the compounds, xenaldial was able to significantly decrease the intracellular and extracellular viral content. More precisely, it was observed that the virus released by the cells appeared in the fluid with a 24 hour delay compared with controls, and that the intracellular virus amount was reduced compared with the control by above one logarithmic unit up to 72 hours after the infection. Subsequently, the virus content in culture fluids can be considered comparable to that of the control, as a consequence of the progressive deactivation of the compound. It has however been observed that a further administration of xenaldial 48 hours after the infection once again remarkably reduced the intra- and extra-cellular virus amount up to 120 hours.

On the whole, the experimental results confirm that xenaldial is able to exert a virucidal action against the HIV virus as well as an inhibiting activity on the reproductive mechanism of said virus. More particularly, xenaldial proved to interfere with the intracellular stages of the viral biological cycle. Although the molecular mechanism has not yet completely been elucidated, the inhibitory effect exerted by this drug on HIV growth is likely due to inhibiting activity on integrase. In any event, the invention is not bound by the action mechanism of the compounds with anti-HIV activity herein disclosed.

As for xenalic acid and xenalamine, they were found to exert a marked virucidal activity on extracellular viral particles, while their inhibitory effect on intracellular viral replication was lower than that of xenaldial under the same experimental conditions.

Therefore, xenaldial, xenalamine and xenalic acid may be used in the treatment of HIV infections and related conditions, particularly AIDS. Furthermore, they may be used in the treatment of HIV infection related conditions, such as ARC ("AIDS related complex"), both symptomatic and asymptomatic, or when exposure to HIV virus is suspected.

For the use in therapy, the compounds will be suitably formulated with pharmaceutically acceptable excipients and carriers. Suitable forms for the oral, parenteral or inhalatory administrations will be, for example, capsules, powders, granules, suppositories, solutions, suspensions, syrups, emulsions, injectable solutions, spray solutions or suspensions. The pharmaceutical compositions will be formulated according to conventional techniques, as described, for example, in Remington's Pharmaceutical Sciences Handbook, Mack Pub. Co., NY, USA, XVII Ed.

Dosages will vary depending on the severity of the disease, the general conditions and age of the patient, and will usually range from 1 to 1,000 mg of compound per Kg body weight, in single or multiple dosages, preferably from 1 to 100 mg/Kg.

The compounds of the invention may also be used in combination with other anti-HIV treatments currently available. By way of example, HIV protease inhibitors, such as saquinavir, indinavir, ritonavir, nelfinavir; reverse transcriptase nucleoside and non-nucleoside inhibitors, such as azidothymidine, dideoxycytidine and dideoxyinosine, or other integrase inhibitors (see e.g. *10*).

Therefore, the invention also relates to pharmaceutical compositions in the form of combined preparations, for the simultaneous, separated or sequential use in the treatment of HIV infections, comprising a compound selected from the group of xenaldial, xenalamine and xenalic acid, and at least on anti-HIV agent selected from the group of protease inhibitors, reverse transcriptase nucleoside or non-nucleoside inhibitors, integrase inhibitors.

The following example illustrates the invention in greater detail.

### EXAMPLE 1

### Materials

Lymphoblasts cultures in stationary tubes (inoculum 200,000 cells/ml) at the 4^{th} day from implant were used. A lactalbumin medium enriched with 20% decomplemented calf serum medium was used as culture medium (TC) and, as maintaining medium (TM), after infection of the cultures, a 199 supplemented with 1% bovine crystallized albumin and 2% calf serum was used. All media were added with penicillin (100 U.I/ml) and streptomycin (100 µg/ml). Viral dilutions were carried out with 199 only.

### Drugs

Xenalamine (p-α-ethoxy-p-phenyl-phenacylaminobenzoic acid), xenalic acid (p-(4-biphenyl-glyoxylidene)-amino-benzoic acid) at a 12.5 µg dose; xenaldial (4,4'-bis-diphenylglyoxal hydrate) at a 25 µg dose.

The dose used for each drug is the maximal dose tolerated by the cultures, established in previous experiments.

The compounds were dissolved in Carbowax 200, heating to 100°C for 15 min.

### General scheme of the antiviral activity assay

9.9 ml of broth containing the virus in the form of a suspension diluted to 10⁻¹ were added with 0.10 ml of Carbowax with or without chemotherapeutics (xenaldial 2 mg, final concentration 200 µg/ml; xenalamine 23 mg final concentration 230 µg/ml).

The resulting suspensions were incubated at 37°C for an hour while stirring repeatedly.

After incubation, for each suspension a scalar dilution log.₁₀ in broth was carried out. Subsequently, the suspensions were inoculated in lymphocytes cultures (0.10 ml), in groups of 8 tubes per dilution, to evaluate the LD₅₀ for each tested material.

Results are reported in the Table (the indicated numbers are the mean of 3 experiments).

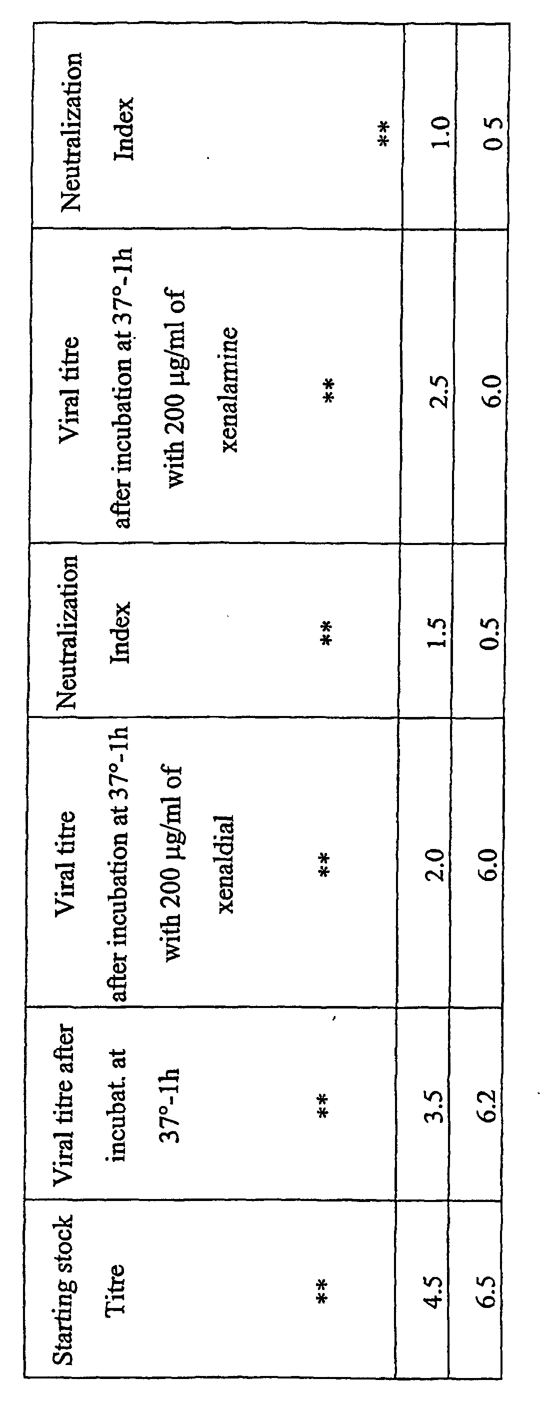

The virus-deactivating action exerted by diphenyl ketoaldehyde derivatives varies between 1 and 1.5 U log.₁₀, and it is slightly more evident with xenaldial than with xenalamine. Under the used experimental conditions, already marked reductions of the viral titre (1 U log.₁₀) and of the LD₅₀ after exposure for 1 hour at 37°C are observed (compared with the control suspensions).

The Table evidences that the virus is poorly sensitive in vitro to the direct deactivating action of the tested compounds.

The reduction of the pathogenic activity of the virus upon exposure to the compounds is as follows (expressed as % of the logarithmic values compared with controls):
xenaldial- low viral titre 42.8%;
xenaldial - high viral titre 8.3%;
xenalamine - low viral titre 28.5%;
xenalamine- high viral titre 8.3%.

### Procedure

The following procedure was used to evaluate the viral growth curve in the presence of the tested compounds: cultures were infected with 1 ml of viral fluid containing a cytopathogenic dose to obtain an about 1:1 LD₅₀/cell ratio. 1 Hour after the infection at +36°C and repeated washings with Hanks balanced saline solution, the viral fluid was replaced with TM for the control groups and with TM + drug for the tested cultures.

At time intervals, aliquots from three cultures were taken, washed with Hanks solution, then added with 1 ml of TM and frozen at -30°C.

At the end of the experiment, the culture fluids were thawed and refrozen twice, pooling each aliquot, then titred simultaneously with the corresponding fluids separated before freezing.

### Results

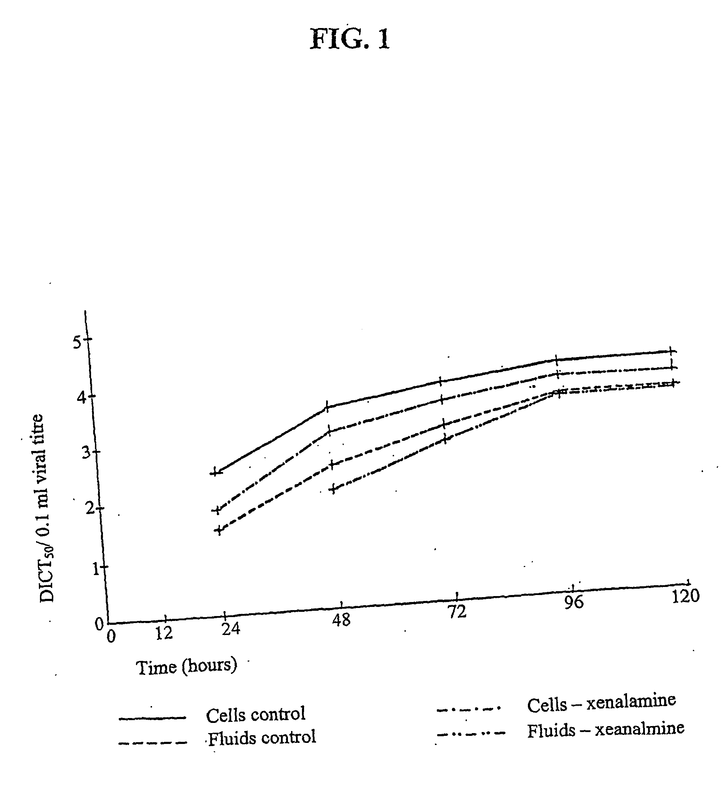

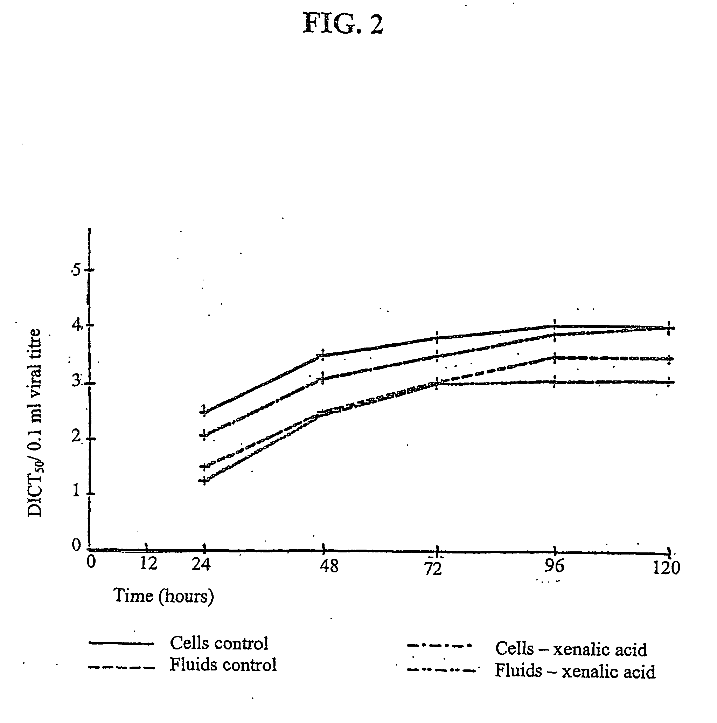

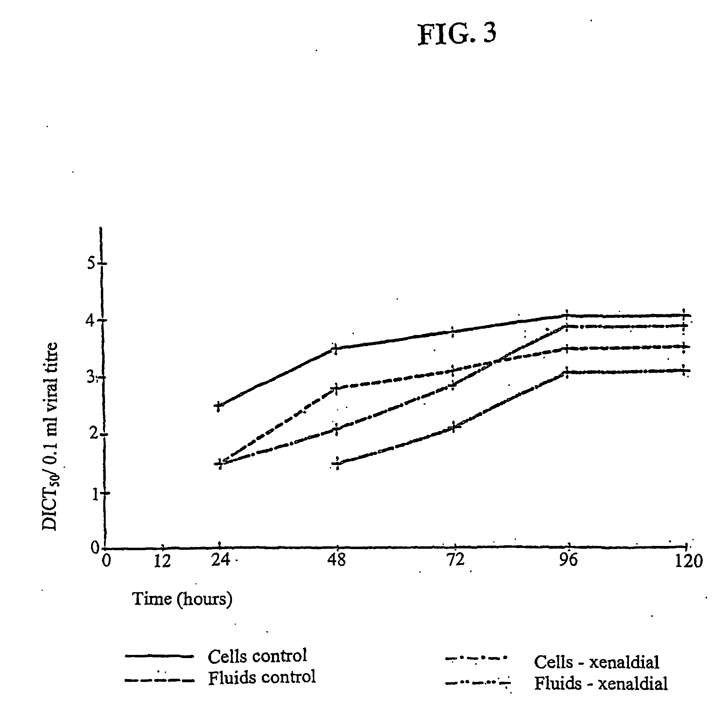

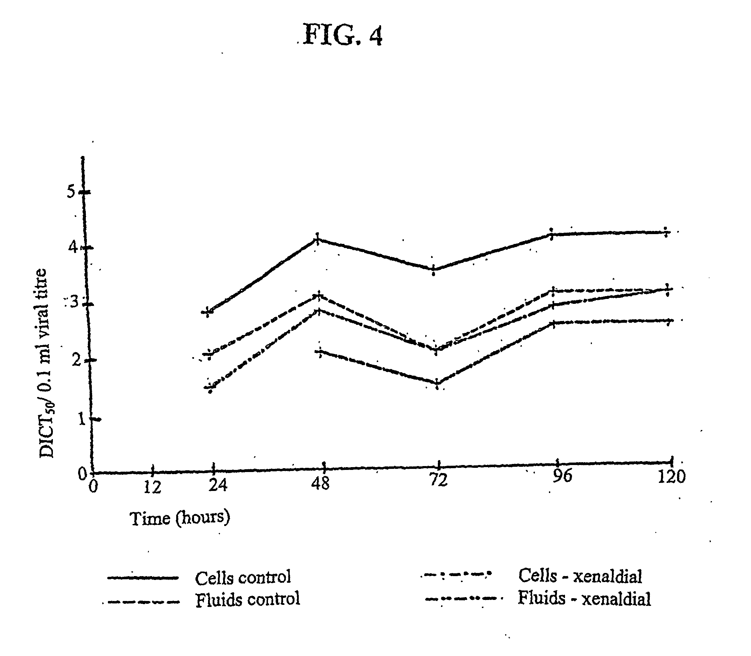

Results are plotted in Figures 1-4, where:
Fig. 1: viral growth curve in the presence of xenalamine, added to the cultures 1 h after the infection at a dose of 12.5 µg/ml;
Fig. 2: viral growth curve in the presence of xenalic acid, added to the cultures 1 h after the infection at a dose of 12.5 µg/ml;
Fig. 3: viral growth curve in the presence of xenaldial, added to the cultures 1 h after the infection at a dose of 25 µg/ml;
Fig. 4: viral growth curve in the presence of xenaldial, added to the cultures 1 h and 48 hours after the infection at a dose of 25 µg/ml.

The plots show:
- in cultures treated with xenalamine, an about 24 hours delay in the appearance of the virus in fluids;
- in cultures treated with xenaldial, significant changes in the intracellular and extracellular viral content; the intracellular virus amount is lower than the control by more than 1 U log up to 72 hours; afterwards, the reduction progressively decreases until disappearing at the end of the experiment. The virus released from the cells appears in fluids with a 24 hour delay compared with controls. When, 48 hours after the infection, the culture medium is replaced with 1 ml of TM containing 25 µg/ml xenaldial, a reduction of the intra- and extracellular virus amount up to 120 hours compared with controls is observed.

### EXAMPLE 2

HTLV IIIB-inhibitory activity of Xenaldial was tested on cultures of lymphocytes. The % virus inhibition was determined by means of PCR, according to the following procedure:

### DNA preparation

DNA was extracted from 5x10⁴ lymphocytes using 400 µl lysate and incubating at 56°C/60' and at 92°C/15'. Afterwards the mixture was cooled at room temperature and kept at 4°C.

### Probe

The oligonucleotide SK19 (41 bases - corresponding to the internal sequence of the amplified pro-viral DNA) was synthesised, dissolved in water at a final concentration of 20 µm and 5'-labelled with polynucleotide kinase and ³²P-ATP.

### PCR

Reagents: Taq polymerase 5U/µl, deoxynucleotides (10 mM pH7.0), KCl 2M, Tris-Cl 1M pH 8.3, MgCl2 1M, Gelatine, primers 15 µM, probe 15 µM, ³²P-ATP, Polynucleotide kinase 10U/µl, DNA 1-3 µg, EDTA 0.25M, proteinase K 10 mg/ml, SDS 10%, ammonium acetate 2M, SSC 20x, TRIS-borate-EDTA 10x, TRIS-acetate-EDTA 10x, Loading buffer 6x (blue bromophenol 0.25%, xylen-cyanol 0.25%. sucrose 40%, TRIS 10 mM pH 8.0), lysate mixture (K-proteinase 60 µg/ml, NP-40 0.5%, Tween 20 0.5%, TRIS 5 mM pH 8.3, MgCl2 1.25 mM, KCl 50 mM);

Reagent preparation: Nucleotides: 125 µl of each nucleotide 10 mM were diluted to 1ml with H2O (final concentration 1.25 mM); buffer 10x: KCl 2M - 250 µl, TrisCl 1M pH8.3 - 100 µl, MgCl2 1M - 20 µl, gelatine - 10 mg, diluted to 1 ml with H2O;

Oligonucleotide pair: oligonucleotides SK38 and 39 were synthesised, purified and dissolved in a concentration of 200-300 µM.

Reaction: mixing buffer 10x, nucleotides (1.25 mM), oligonucleotide primer SK38 and SK39 15 µM, lysate, Taq polymerase 5 U/µl; denaturation 90°C/60', annealing 55°C/30', polymerisation 72°C/30'; repeat the cycle 30 times.

The results are reported in the following Tables:

| XENALDIAL 0.001-5 µM - HTLV IIIB | | | |
|---|---|---|---|
| Drug concentration-µM | Mean OD | Mean OD corrected by dilution | Inhibition % |
| Virus without drug | 3740 | 374000 | - |
| 0.001 | 2096 | 209600 | 44% |
| 0.01 | 2654 | 265400 | 30% |
| 0.1 | 3300 | 330000 | 12% |
| 1 | 3144 | 314400 | 16% |
| 5 | 3630 | 363000 | 42% |

| XENALDIAL 10-100 µM - HTLV IIIB | | | |
|---|---|---|---|
| Drug concentration-µM | Mean OD | Mean OD corrected by dilution | Inhibition % |
| Virus without drug | 2610 | 261000 | - |
| 10 | 3948 | 394800 | - |
| 20 | 1941 | 194100 | 26% |
| 40 | 3324 | 332400 | - |
| 60 | 1094 | 109400 | 59% |
| 100 | 610 | 61000 | 77% |

### REFERENCES

*1.* Characterization of HIV-1 Integrase Inhibitors, M.S.T. HANSEN et al., The Salk Inst. For Biological Studies, La Jolla, CA.
*2.* Transduction Efficiency and Gene Expression from Integrase-Defective Lentiviral Hybrid-Vectors Containing an Episomal Origin Of Replication, J. VARGAS, et al., Mt. Sinai Sch. Of Med., New York, N.Y.
*3.* The HERV-K Proviruses in All Humans, M. BARBULESCU, et al., Albert Einstein Coll. Of Med., Bronx, N.Y. and Yale Univ. Sch. Of Med., New Haven, CT.
*4.* A Novel Inducible System To Study HIV-1 Vpr Function, Y.ZHOU and L. RATNER, Washington Univ. Sch. Of Med., St. Louis, MO.
*5.* Functional Implications of the Interaction of HIV-1 Vpr with a Fission Yeast Orthologue (Rhp23) of the Human Nucleotide Excision Repair Gene HHR23A, R.T. ELDER, et al., Children's Mem. Inst. For Ed. and Res., Northwestern Univ, Med. Sch. Chicago, IL.
*6.* Nuclear Import and Export of HIV-1 Vpr, M. P. SHERMAN, et al., Gladstone Inst. of Virology and Immunology and Univ. of California, San Francisco.
*7.* Inhibitors of HIV Integrase: Antiviral Activity and Mechanism. D.J. HAZUDA. Merck Res. Labs, West Point, PA.
*8.* Chemoterapia 9, 1964, 6:341-358.
*9.* Lorenzutti, G., et al.: Studio comparative di alcuni derivati chetoaldeidici in rapporto alla loro azione inattivante in vitro sul virus Influenzale A-PR8 Boll. Soc. ital. Biol. Sper. 40:1221 (1964).
*10.* Science, 2000, Vol. 287:646-650.

## Claims

1. The use of a compound selected from xenalamine, xenalic acid and xenaldial for the preparation of a medicament for the treatment of HIV infections.

2. The use of xenaldial as claimed in claim 1.

3. The use as claimed in claim 1 or 2, for the prophylaxis or the therapy of AIDS and related diseases.

4. Pharmaceutical compositions containing an effective amount of a compound as claimed in claim 1 together with a different anti-HIV agent.

5. Compositions according to claim 4, wherein the anti-HIV agent is selected from protease inhibitors, reverse transcriptase inhibitors and integrase inhibitors.

## Patentansprüche

1. Verwendung einer Verbindung, ausgewählt aus Xenalamin, Xenalin-Säure und Xenaldial, zur Zubereitung eines Medikaments zur Behandlung von HIV-Infektionen.

2. Verwendung von Xenaldial, wie in Anspruch 1 beansprucht.

3. Verwendung wie in Anspruch 1 oder 2 beansprucht, zur Prophylaxe oder Therapie von AIDS und verwandten Krankheiten.

4. Pharmazeutische Zusammensetzungen, die eine wirksame Menge einer Verbindung, wie sie in Anspruch 1 beansprucht ist, gemeinsam mit einem anderen Anti-HIV-Wirkstoff enthalten.

5. Zusammensetzungen gemäß Anspruch 4, bei denen der Anti-HIV-Wirkstoff ausgewählt ist aus Protease-Inhibitoren, Inhibitoren reverser Transkriptase und Integrase-Inhibitoren.

## Revendications

1. Utilisation d'un composé choisi parmi la xénalamine, l'acide xénalique et le xénaldial pour la préparation d'un médicament pour le traitement des infections par VIH.

2. Utilisation du xénaldial telle que revendiquée dans la revendication 1.

3. Utilisation telle que revendiquée dans la revendication 1 ou 2, pour la prophylaxie ou la thérapie du SIDA et des maladies associées.

4. Compositions pharmaceutiques contenant une quantité efficace d'un composé tel que revendiqué dans la revendication 1 conjointement avec un agent anti-VIH différent.

5. Compositions selon la revendication 4, dans lesquelles l'agent anti-VIH est choisi parmi les inhibiteurs de protéase, les inhibiteurs de transcriptase réverse et les inhibiteurs d'intégrase.
